(19) Europäisches Patentamt
European Patent Office
Office européen des brevets

(11) Numéro de publication : **0 051 613**
**B1**

(12) **FASCICULE DE BREVET EUROPÉEN**

(45) Date de publication du fascicule du brevet :
04.07.84

(51) Int. Cl.³ : **A 61 B 3/04**, G 02 C 7/08

(21) Numéro de dépôt : **81901134.7**

(22) Date de dépôt : **05.05.81**

(86) Numéro de dépôt international :
**PCT/FR 81/00059**

(87) Numéro de publication internationale :
**WO/8103119 (12.11.81 Gazette 81/27)**

(54) **DISPOSITIF OPTIQUE CORRECTEUR PROVISOIRE PERMETTANT LE CHOIX D'UNE MONTURE NUE.**

(30) Priorité : 08.05.80 FR 8010669

(43) Date de publication de la demande :
19.05.82 Bulletin 82/20

(45) Mention de la délivrance du brevet :
04.07.84 Bulletin 84/27

(84) Etats contractants désignés :
**DE**

(56) Documents cités :
**FR-A- 616 140**
**FR-A- 822 592**
**FR-A- 1 033 523**

(73) Titulaire : **L.H.P. LOUPES HAUTES PERFORMANCES**
**43, rue René-Leynaud**
**F-69001 Lyon (FR)**

(72) Inventeur : **RETHORE, Gérard**
**4, Grande Rue**
**F-34200 Sète (FR)**

(74) Mandataire : **Ecal, François**
**4, rue Fabrégat**
**F-34500 Béziers (FR)**

Jouve, 18, rue St-Denis, 75001 Paris, France

## Description

La présente invention a pour objet des dispositifs selon la revendication 1, permettant à la clientèle des opticiens de choisir la monture nue la plus seyante à son visage avant qu'y soient montés des verres correcteurs.

Un tel choix est difficile pour la clientèle, les futures montures des lunettes correctrices étant évidemment essayées nues de toute optique. De sorte que le le client se trouve avoir une grande difficulté pour exécuter ce choix, ne voyant que très mal l'objet dont il chausse son nez. Ou bien il met ses propres lunettes qu'il surmonte de la prochaine monture, ce qui nuit à la connaissance exacte de l'esthétique de cette dernière.

Il existe cependant des dispositifs permettant une correction suffisante de la vue du client et pouvant être adjoints aux lunettes essayées, afin que le client qui en est muni puisse effectuer son choix en connaissance de cause.

Un tel dispositif connu se présente parfois sous la forme d'une monture légère, de très faible surface, munie de verres correcteurs de petit diamètre qui y sont scellés et qui présentent un écartement moyen des centres optiques. Cette paire de lunettes transitoire possède un bord tombé supérieur qui permet de la suspendre provisoirement à une paire de lunettes quelconque en la posant sur sa partie supérieure.

Muni d'un tel dispositif qui se superpose à la paire de lunettes nue essayée, le client peut voir l'effet d'esthétique produit par celle-ci.

Mais d'une part ce dispositif est particulièrement instable, se décrochant facilement des lunettes qui le portent. Mais en outre, du fait précisément de son exiguité, et du caractère hétérogène de ses composants, il forme un objet aux contours multiples et visibles qui s'inscrit dans le cadre des lunettes essayées et qui de ce fait gêne l'observation de leur esthétique.

Selon un autre dispositif connu l'élément correcteur provisoire est constitué par un verre correcteur isolé de petit diamètre muni à sa périphérie de fines lames de ressorts tangentiels, qui par leur élasticité permettent de maintenir de façon transitoire ledit verre correcteur dans la lunette d'une nouvelle monture, lesdits ressorts pénétrant dans le drageoir. Un tel dispositif se montre dans chacune des lunettes de la nouvelle paire à essayer.

Ici encore il est évident que le dispositif de ressorts qui se trouve à l'intérieur de chaque lunette gêne particulièrement l'observation de la paire que l'on essaye.

D'autre part dans les deux cas de tels dispositifs ne peuvent convenir pour toute la gamme des corrections nécessaires. En effet l'opticien qui procède à ces essais ne peut pas posséder une série de dispositifs correcteurs suffisamment fournie pour satisfaire la vue de tous ses clients. Les constructeurs de ces dispositifs ne fournissent d'ailleurs qu'une gamme restreinte de correction. Or aucun de ces deux dispositifs ne permet la superposition de deux ou plusieurs organes correcteurs pour atteindre la puissance parfois nécessaire. Dans le premier cas la superposition de deux dispositifs ne permettrait pas la coïncidence en hauteur des axes optiques, puisqu'ils sont posés l'un sur l'autre par leur cornière supérieure. Dans le second cas il y a impossibilité matérielle d'insérer dans un même drageoir plusieurs dispositifs à ressorts.

Le dispositif selon la revendication 1, objet de l'invention, a pour but d'éviter ces inconvénients. Celui-ci en effet permet une superposition parfaite de plusieurs optiques provisoires afin de disposer de la puissance désirée, les axes optiques restant toujours parfaitement alignés.

De même la surface de ce dispositif est telle qu'au lieu d'être inscrite dans la monture à essayer, comme le sont les dispositifs connus, elle est au contraire très largement circonscrite aux lunettes essayées et parfaitement transparente, de telle manière que le client peut voir la monture qu'il essaye à travers une surface correctrice dont il ne distingue pas les limites qui viendraient dénaturer l'esthétique de la monture essayée. D'autant plus que la zone correctrice dudit dispositif provisoire étant taillée dans la même masse que sa monture, il n'y a au niveau de la périphérie propre pratiquement aucune rupture apparente de continuité qui provoquerait l'apparition d'un cerne gênant, comme cela est dans les dispositifs connus.

Les dessins annexés, donnés à titre d'exemple seulement, montrent un mode de réalisation des dispositifs objet de la présente invention.

La figure 1 est une vue schématique en plan du dispositif objet de l'invention selon un mode de réalisation.

La figure 2 est une vue schématique en coupe selon AB dudit dispositif, à plus grande échelle.

La figure 3 est une vue schématique montrant l'utilisation du dispositif.

La figure 4 est une vue schématique en plan du dispositif objet de l'invention selon un autre mode de réalisation.

La figure 5 est une vue schématique en coupe selon CD du dispositif ci-dessus, à plus grande échelle et partielle.

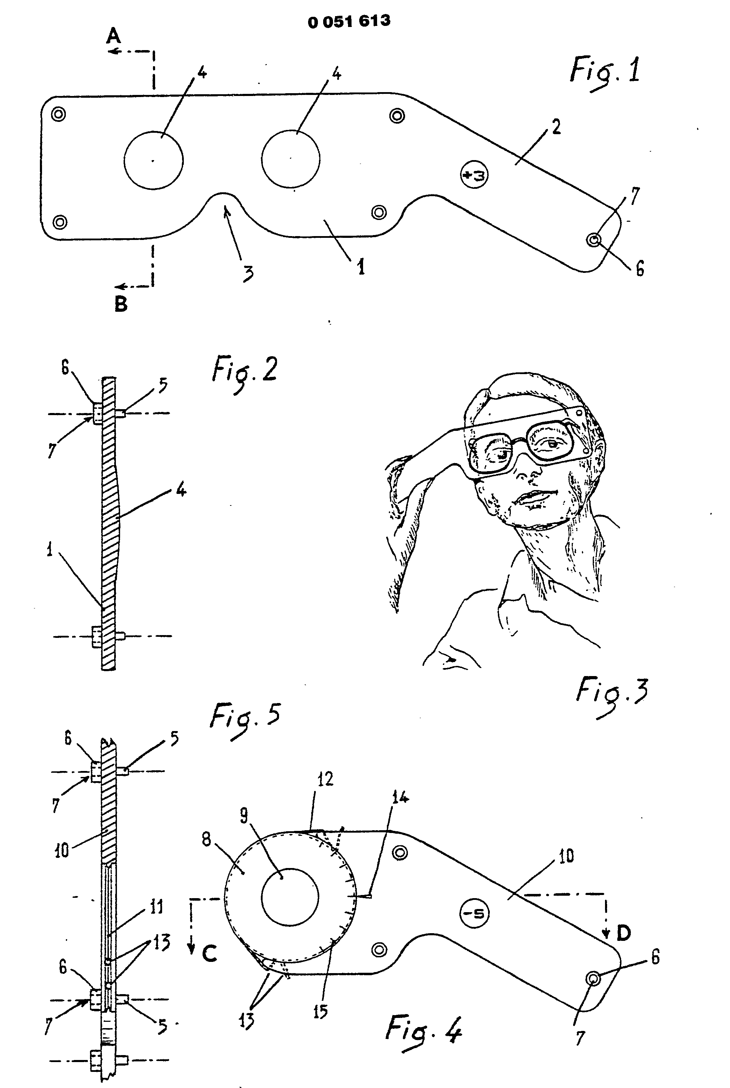

Tel qu'il est représenté le dispositif est constitué par une plaque de matière rigide parfaitement transparente (1), telle que le métacrylate de méthyle, qui est découpée selon une forme géométrique simple circonscrivant très largement la surface d'une monture de lunettes et comportant sur un côté un manche (2) formé de la même pièce, servant à la prise de l'objet. Une échancrure (3) est prévue à la base de ladite plaque pour situer la position du nez et par le fait même centrer optiquement l'appareil, dans la masse duquel ont été travaillées (par injection dans un moule adéquat par exemple) les deux surfaces correctrices (4), à l'écartement nécessaire, cette méthode permettant de ne pas laisser pratiquement apparaître de cerne à la limite des surfaces correctrices, comme cela serait le cas si elles étaient faites d'une matière transparente différente qui ne présenterait pas le même indice de réfraction que la plaque-monture. Enfin (figure 2) le dispositif est muni sur l'une de ses faces de au moins trois tétons (5) et sur l'autre face, dans le même axe que lesdits tétons, d'autant de saillies (6) munies chacune d'une cuvette (7) pouvant recevoir lesdits tétons d'une plaque voisine. Ainsi chaque plaque peut être posée sur un meuble sur l'une quelconque de ses faces, sans que sa surface puisse être rayée, étant éloignée dudit meuble par les tétons (5) ou les saillies (6). Et les tétons (5) d'une plaque pouvant pénétrer dans les cuvettes (7) d'une plaque voisine, il est rendu facile de combiner plusieurs plaques qui se trouveront ainsi parfaitement alignées et immobilisées entre elles. De sorte qu'avec un nombre restreint de plaques présentant une gamme de puissance standard il est possible de les conjuguer entre elles pour obtenir une gamme très étendue de puissance s'adaptant à la vue de l'ensemble de la clientèle.

Le dispositif étant ainsi constitué on comprend qu'il sera facile, pour le client qui désire essayer une nouvelle monture de lunettes non encore équipée de ses verres correcteurs, de prendre en main une ou plusieurs plaques associées comme il vient d'être dit, selon la correction nécessaire, et de les saisir comme un face à main placé à l'avant de ladite monture, pour voir nettement celle-ci dans une glace sans que le contour pratiquement inapparent du dispositif trouble l'esthétique recherchée, la paire de lunettes observée s'inscrivant très en deçà des limites extérieures du dispositif qui par sa parfaite transparence n'offre au surplus qu'une très légère vue de son contour extérieur.

On remarquera de plus que pour parfaire la correction, il est facile de placer le dispositif à une distance variable du plan oculaire, sans rien gêner pour l'observation des lunettes essayées, ce qui n'est pas possible avec les dispositifs connus qui ont pour seul support la paire de lunettes elle-même.

Accessoirement il est indiqué que la puissance optique de chaque plaque correctrice est gravée de façon très légère sur le manche de celle-ci.

Selon un autre mode de réalisation (fig. 4) la plaque transparente (8) du support de la surface optique (9) épouse une forme parfaitement circulaire circonscrivant très largement la surface d'une lunette, ladite plaque transparente circulaire pouvant être tenue en main au moyen du manche (10) fait de la même matière, qui permet de présenter le dispositif comme un face à main monoculaire.

Une telle présentation du dispositif objet de l'invention permet de l'orienter de différentes manières par rapport à son centre optique, ce qui permet de l'utiliser pour faciliter l'observation des nouvelles montures par les astigmates, la surface correctrice étant prévue dans ce cas en conséquence.

Dans ce même but, et pour rendre son emploi encore plus aisé, la plaque circulaire (8) porteuse de la surface correctrice (9) est rendue mobile par rapport à son manche (10) de façon à pouvoir être orientée selon l'angle d'astigmatisme du sujet, sans qu'il soit nécessaire de modifier la position du manche, la liaison entre eux étant obtenue à l'aide d'un dispositif aussi peu apparent que possible.

Pour cela le manche (10) (fig. 5) présente une partie incurvée formant berceau destiné à recevoir la plaque optique (8) sur sa tranche. Et ce berceau lui-même est muni d'une fine arête aiguë (11) qui vient se loger dans le léger drageoir pratiqué à la périphérie de la plaque optique (8). Ladite plaque optique est alors maintenue contre ledit berceau au moyen d'un fil de matière plastique (12) résistant qui est naturellement arrêté de part et d'autre dudit berceau en passant successivement dans les doubles perforations (13) qui sont pratiquées à chacune des extrémités du berceau.

La tension du fil (12) est telle que la plaque optique (8) se trouve solidaire du manche (10) grâce à ce fil qui parcourt le drageoir périphérique. Mais sa tension permet la rotation à frottement doux de la plaque (8) autour de son centre.

On comprend donc que, si la surface optique centrale est conçue avec une cylindricité connue, son axe pourra être orienté selon les différents angles nécessaires pour corriger l'astigmatisme des usagers. Pour faciliter le calage angulaire du dispositif un index (14) est finement gravé sur le manche et des repères (15) sont aussi finement gravés sur la périphérie de la plaque (8).

On comprend donc qu'ainsi le dispositif est adaptable aux astigmates, en conservant ses qualités de transparence et d'absence de limite visible qui détruirait l'harmonie de la monture de lunettes devant laquelle elle est placée et à travers laquelle elle est observée.

Il est bien entendu que l'étendue de l'invention n'est pas limitée aux exemples qui en ont été donnés, mais inclut toute variante considérée comme équivalente dans le cadre des revendications.

## Revendications

1. Dispositif optique correcteur de vision destiné à être utilisé de façon provisoire pour l'observation dans tous miroirs, par le client lui-même, de l'ensemble de sa personne s'il le désire, en vue d'apprécier l'esthétique d'une monture de lunettes qu'il essaye et qui est encore dépourvue des verres correcteurs définitifs capable de lui assurer la même vision générale, caractérisé par le fait qu'il comporte un support permettant au client de placer en avant de la monture de lunettes dépourvue de verres qu'il essaye et à la distance convenable pour la correction de sa vue un dispositif correcteur de vision provisoire compatible avec sa vue mais ne comportant lui-même aucune monture qui viendrait se superposer à la monture qu'il cherche à observer et en rendrait de ce fait l'observation impossible ou pour le moins troublée, ledit support étant constitué par une plaque rigide généralement parfaitement plane et parfaitement transparente dont les limites circonscrivent largement la surface de la monture à observer, ladite plaque généralement plane comportant dans les axes optiques des yeux, ménagées dans sa masse et sans qu'aucun dénivellement brusque soit observable, les formes convergentes ou divergentes nécessaires à la correction de la vue du client considéré, lesdites surfaces optiques correctrices ainsi créées n'étant de ce fait matériellement définies par aucun contour apparent.

2. Dispositif selon la revendication 1, caractérisé par le fait qu'il comporte, découpé dans la même matière parfaitement transparente, et sans être serti dans aucun cadre hétérogène, de manière à être aussi le plus inapparent possible, un manche qui permet à l'utilisateur de placer ledit dispositif devant ses yeux à la manière d'un face à main.

3. Dispositif selon la revendication 2, caractérisé par le fait que lorsqu'il est monoculaire, la plaque rigide transparente (8), qui constitue le support de la surface optique (9), est de forme circulaire, d'un diamètre très supérieur à celui de la lunette qu'il recouvre, et comporte sur la périphérie de sa tranche une rainure capable de recevoir un fil plastique (12) résistant qui maintient le disque (8) ainsi formé serré contre le manche (10) qui épouse, dans sa partie tangente au disque (8), une forme d'arc de cercle femelle de même diamètre que ce dernier et qui est munie à mi-épaisseur d'une arête aiguë (11) capable de pénétrer dans la rainure dudit disque, le fil plastique (12) qui l'enserre à frottement doux étant arrêté et tendu de part et d'autre dudit arc de cercle qui délimite le manche (10) en ce point ; la surface optique (9) étant prévue dans ce cas pour convenir à la correction des astigmates ; de légers repères (15) faits sur la périphérie du disque (8) permettant le repérage de l'angle donné au disque, donc à la surface optique, pour corriger provisoirement l'astigmatisme de l'usager pour lui permettre d'observer la monture qu'il essaye et devant laquelle ledit dispositif est provisoirement placé.

4. Dispositif selon la revendication 2, caractérisé par le fait que, lorsqu'il est binoculaire, la plaque rigide transparente qui supporte les surfaces optiques et qui est elle-même d'une surface très supérieure à la surface de la paire de lunettes, comporte à sa base une échancrure (3) qui permet de la placer sur le nez de l'utilisateur, ce qui situe les surfaces optiques (4) en coïncidence avec les axes optiques de ses yeux.

5. Dispositif selon l'une quelconque des revendications 3 ou 4, caractérisé par le fait qu'il comporte sur une de ses faces trois tétons (5) au moins, faits de la même matière transparente, capables de tenir la surface dudit dispositif écartée de tout meuble sur lequel il serait posé ; et comporte sur l'autre face et selon les mêmes axes des saillies (6) faites de la même matière transparente capables de jouer le même rôle de pieds, lesdites saillies étant munies de cuvettes (7) capables de recevoir les tétons (5) d'une plaque voisine qui serait adjointe à la première de manière à parfaitement immobiliser plusieurs plaques entre elles dans la parfaite coïncidence des axes optiques, en vue de combiner leur pouvoir correcteur pour l'ajuster à la vue du client.

**Claims**

1. A vision-corrective optical device intended for being employed temporarily for observation by the client himself in any mirror, of the whole of his person if he so desires, with a view to appreciating the aesthetic of a spectacle frame which he is trying out and which still lacks definite corrective lenses capable of ensuring him the same general vision, characterized by the fact that it includes a support enabling the client to place in front of the unglazed spectacle frame which he is trying out and at the distance suitable for the correction of his sight a temporary vision-corrective device compatible with his sight but not itself having any frame which would become superimposed upon the frame which he is trying to observe and consequently make observation impossible or at least impeded, the said support consisting of a rigid plate which in general is perfectly plane and perfectly transparent and of which the edges broadly circumscribe the area of the frame being observed, the said generally plane plate including on the optical axes of the eyes and arranged in the body of it the convergent or divergent shapes necessary to the correction of the sight of the client in question, the said corrective optical areas so created consequently not being defined by any visible outline.

2. A device as in Claim 1, characterized by the fact that it includes, cut out from the same perfectly transparent material and without being set in any sort of frame, so as to be also as invisible as possible, a handle which enables the user to place the said device in front of his eyes after the style of a lorgnette.

3. A device as in Claim 2, characterized by the fact that when it is for only one eye the rigid transparent plate (8) which forms the support of the optical area (9) is circular in shape and of much greater diameter than that of the lens which it is covering, and includes along the periphery of its edge a groove capable of receiving a strong plastics thread (12) which holds the disc (8) so formed tight against the handle (10) which along the portion of it touching the disc (8) adopts a female shape of an arc of a circle of the same diameter as the disc, and which is equipped in mid-thickness with a sharp ridge (11) capable of entering the groove in the said disc, the plastics thread (12) which encloses it with light friction being fastened and stretched at opposite ends of the said arc of a circle which bounds the handle

(10) at this point; the optical area (9) being designed in this case to suit correction of astigmatics; light markings (15) made on the periphery of the disc (8) enabling marking of the angle given to the disc and hence to the optical area in order to correct the user's astigmatism temporarily in order to enable him to observe the frame which he is trying out and in front of which the said device is temporarily placed.

4. A device as in Claim 2, characterized by the fact that when it is for both eyes the rigid transparent plate which supports the optical areas and which is itself of an area very much larger than the area of the pair of spectacles, includes at the bottom of it a notch (3) which enables it to be placed on the user's nose, which locates the optical areas (4) in coincidence with the optical axes of his eyes.

5. A device as in either of the Claims 3 or 4, characterized by the fact that it includes on one of its faces at least three studs (5) made of the same transparent material and capable of keeping the surface of the said device away from any piece of furniture upon which it might be laid, and includes on the other face and along the same axes projections (6) made of the same transparent material and capable of playing the same part of feet, the said projections being equipped with cups (7) capable of receiving the studs (5) on an adjacent plate which might be added to the first so as to fix a number of plates perfectly together in perfect coincidence of the optical axes, with a view to combining their corrective power in order to adjust it to the sight of the client.

**Ansprüche**

1. Vorrichtung zur provisorischen optischen Sichtkorrektur zwecks Betrachtung der Gesamtheit seiner Person, wenn er dies wünscht, durch den Kunden selbst in allen Spiegeln im Hinblick auf die Beurteilung der Ästhetik eines Brillengestells, das er anprobiert und das noch keine definitiven Korrekturgläser aufweist, die in der Lage wären, ihm die gleiche umfassende Sicht zu gewährleisten, gekennzeichnet durch eine Halterung, die es dem Kunden ermöglicht, vor das noch keine Gläser aufweisende Brillengestell, das er anprobiert, und in für die Sichtkorrektur passendem Abstand eine provisorische Sichtkorrektureinrichtung zu halten, die aber selbst kein Gestell besitzt, das sich dem Gestell überlagern könnte, das er anschauen möchte, und die deshalb die Betrachtung unmöglich machen oder zumindest beeinträchtigen würde, wobei diese Halterung aus einer im wesentlichen vollkommen ebenen und vollkommen durchsichtigen, starren Scheibe besteht, deren Ränder das anzuschauende Gestell in weitem Abstand umgeben und die in den optischen Augachsen die zur Sichtkorrektur des infragestehenden Kunden notwendigen konvergenten oder divergenten Formen aufweist, die im Material der Scheibe selber angeordnet sind, so daß die derart geschaffenen optischen Korrekturflächen aus diesem Grund faktisch durch keine sichtbare Kontur materiell definiert sind.

2. Vorrichtung nach Anspruch 1, dadurch gekennzeichnet, daß sie einen Stiel aufweist, der aus demselben vollkommen durchsichtigen Material gestanzt und in keinerlei andersgearteten Rahmen gefaßt ist, um selbst auch möglichst unsichtbar zu sein, und der es dem Benutzer ermöglicht, die genannte Vorrichtung wie ein Lorgnon vor seine Augen zu halten.

3. Vorrichtung nach Anspruch 2, dadurch gekennzeichnet, daß, wenn diese monokular ist, die starre durchsichtige Scheibe (8), die die Halterung der optischen Fläche (9) darstellt, rund ist, mit einem Durchmesser, der sehr viel größer als die Brille ist, die sie bedeckt, und daß sie an der Peripherie eine Nut aufweist, die einen widerstandsfähigen Kunststoffaden (12) aufnehmen kann, der die so geformte Scheibe (8) fest gegen den Stiel (10) drückt, der in seinem die Scheibe (8) berührenden Teil die Matrizenform eines Kreisbogens annimmt mit dem gleichen Durchmesser wie die Scheibe, daß die Form auf halber Dicke mit einem scharfkantigen Grat (11) versehen ist, der in die Nut der genannten Scheibe eingreifen kann, wobei der Kunststoffaden (12), der die Scheibe mit leichter Reibung einschließt, auf beiden Seiten des Kreisbogens, der den Stiel (10) in diesem Punkt begrenzt, befestigt und gespannt ist, daß die optische Fläche (9) in diesem Fall für die Korrektur von Astigmatikern geeignet ist, daß schwache Markierungspunkte (15) auf der Peripherie der Scheibe (8) angebracht sind und es ermöglichen, den der Scheibe bzw. der optischen Fläche gegebenen Winkel zu erkennen, um provisorisch den Astigmatismus des Benutzers zu korrigieren und es ihm zu ermöglichen, das Gestell, das er anprobiert und vor das er die genannte Vorrichtung provisorisch hält, zu begutachten.

4. Vorrichtung nach Anspruch 2, dadurch gekennzeichnet, daß, wenn diese binokular ist, die feste durchsichtige Scheibe, die die optischen Flächen hält und die selbst sehr viel größer als eine Brille ist, an ihrem unteren Ende eine Aussparung (3) hat, wodurch der Benutzer sie auf die Nase setzen kann, was die optischen Flächen (4) mit den optischen Achsen seiner Augen übereinstimmen läßt.

5. Vorrichtung nach einem der Ansprüche 3 oder 4, dadurch gekennzeichnet, daß sie auf einer ihrer Seiten mindestens drei aus demselben durchsichtigen Material hergestellte Spitzen (5) aufweist und es dadurch ermöglicht wird, die Fläche der genannten Vorrichtung von jedem Möbelstück, auf das sie gelegt werden würde, fernzuhalten; sie hat auf der anderen Seite und längs den gleichen Achsen aus dem gleichen durchsichtigen Material gefertigte Vorsprünge (6), die auf die gleiche Weise als Stützfuß dienen, wobei die genannten Vorsprünge mit Ausnehmungen (7) versehen sind, die die Spitzen (5) einer Nachbarscheibe aufnehmen können, die mit der ersten so zusammengefügt wird, daß mehrere Scheiben miteinander in vollkommener Koinzidenz der optischen Achsen unbeweglich zusammengefügt werden, um ihre Korrekturleistung zu kombinieren und diese dem Sehvermögen des Kunden anzupassen.

*Fig.1*

*Fig.2*

*Fig.3*

*Fig.5*

*Fig.4*